(19) Europäisches Patentamt
European Patent Office
Office européen des brevets

(11) Veröffentlichungsnummer: **0 044 422 B1**

(12) # EUROPÄISCHE PATENTSCHRIFT

(45) Veröffentlichungstag der Patentschrift: **19.10.83**

(51) Int. Cl.³: **C 07 D 405/14** // (C07D405/14, 249/12, 303/36)

(21) Anmeldenummer: **81104775.2**

(22) Anmeldetag: **22.06.81**

(54) **Neue Glycidyl-1,2,4-triazolidin-3,5-dione und ein Verfahren zu ihrer Herstellung.**

(30) Priorität: **21.07.80 DE 3027623**

(43) Veröffentlichungstag der Anmeldung: **27.01.82 Patentblatt 82/4**

(45) Bekanntmachung des Hinweises auf die Patenterteilung: **19.10.83 Patentblatt 83/42**

(84) Benannte Vertragsstaaten: **CH DE FR GB IT LI**

(56) Entgegenhaltungen:
**EP-A-0 026 312**
**US-A-3 634 445**
**US-A-3 681 374**
**CHEMICAL ABSTRACTS vol. 61, no. 3, 03-08-1964, Columbus, Ohio, USA, J. BOURDAIS »Heterocyclic series II. New syntheses and physico chemical properties of the 1,2,4-triazolidine-3,5-diones« column 3094-95, abstract no. 3094h**

(73) Patentinhaber: **BAYER AG, Zentralbereich Patente, Marken und Lizenzen, D-5090 Leverkusen 1, Bayerwerk (DE)**

(72) Erfinder: **Giesecke, Henning, Dr., Kalk-Muelheimer Strasse 400, D-5000 Köln 80 (DE)**
Erfinder: **Merten, Rudolf, Dr., Berta-von-Suttner-Strasse 55, D-5090 Leverkusen (DE)**
Erfinder: **Rottmaier, Ludwig, Bergstrasse 85, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Neue Glycidyl-1,2,4-triazolidin-3,5-dione und ein Verfahren zu ihrer Herstellung

Gegenstand der vorliegenden Erfindung sind neue Glycidyl-1,2,4-triazolidin-3,5-dione der allgemeinen Formel I

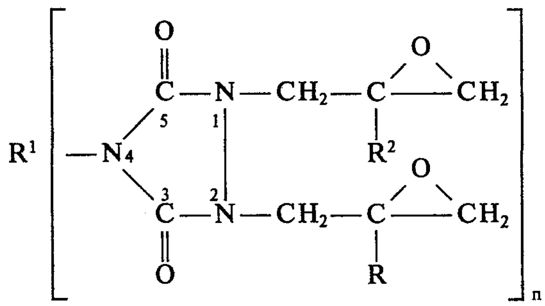

(I)

worin

$R^1$ einen ein- bis fünfwertigen linearen oder verzweigten aliphatischen $C_1-C_{30}$, vorzugsweise $C_1-C_{12}$, einen ein- bis fünfwertigen cycloaliphatischen $C_5-C_{21}$, einen ein- bis fünfwertigen aliphatisch-aromatischen $C_7-C_{17}$, vorzugsweise $C_7-C_{10}$, oder einen ein- bis fünfwertigen aromatischen $C_6-C_{21}$, vorzugsweise $C_6-C_{15}$ Rest, die vorgenannten Reste können durch mindestens eine Alkoxycarbonylgruppe mit $C_1-C_4$ in der Alkoxygruppe, CN, $NO_2$, Alkylmercaptogruppe mit $C_1-C_4$ in der Alkylgruppe, Dialkylaminogruppe mit $C_1-C_6$ in jeder Alkylgruppe, Halogen, und im Falle der aromatischen Reste außer durch die vorgenannten Substituenten durch mindestens eine $C_1-C_4$-Alkylgruppe substituiert sein, ferner können die vorgenannten aliphatischen Reste durch ein oder mehrere Sauerstoffatome oder tertiäre Stickstoffatome, die vorgenannten mehrkernigen cycloaliphatischen, die mehrkernigen aliphatisch-aromatischen und mehrkernigen aromatischen Reste durch mindestens ein Sauerstoff- oder tert. Stickstoffatom oder durch mindestens eine Alkylengruppe mit 1 bis 4 C-Atomen oder durch mindestens eine Sulfonylgruppe

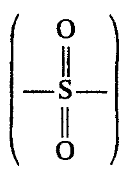

unterbrochen sein,

$R^2$ und $R^3$ gleich oder verschieden für ein Wasserstoffatom oder eine Methylgruppe, vorzugsweise ein Wasserstoffatom, und

n eine Zahl von 1 bis 5, vorzugsweise 1 bis 3,

bedeuten.

Besonders bevorzugt sind Verbindungen der Formel I, in denen $R^1$ unsubstituiert ist und $R^2$ und $R^3$ Wasserstoff bedeuten.

Formelmäßig seien beispielhaft folgende bevorzugte Reste $R^1$ angegeben.

a) $C_nH_{2n+1}-$ n = 1 bis 18

b) $-(CH_2)_m-$ m = 2 bis 12

c) 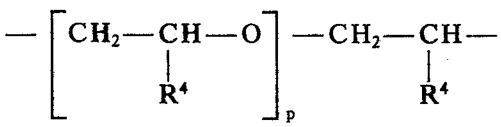 $R^4$ = H, $CH_3$; p = 1–9

d) 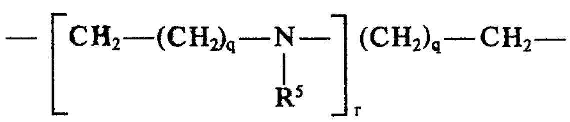 $R^5$ = $C_1-C_4$-Alkyl; q = 1–2; r = 1–4

e) 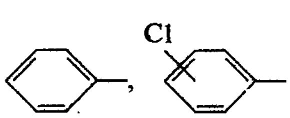

f) 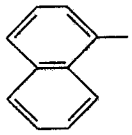

g) 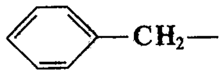

h) 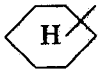

i) 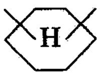

j) 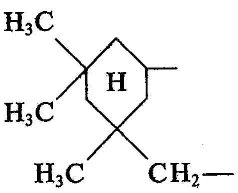

k) 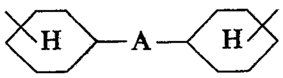

A = Alkylen mit 1–4 C-Atomen, O, $-N(CH_3)-$;

l) 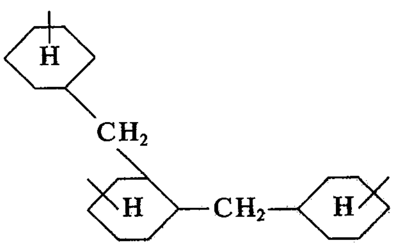

m) 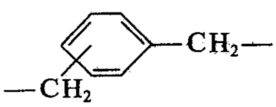

n) 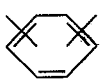

o) 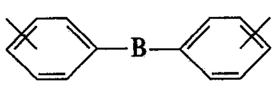

B = Alkylen mit 1–4 C-Atomen, O, $-N(CH_3)-$,

p) 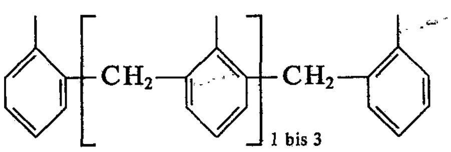

q) $C_4H_9-O-CH_2-CH_2-CH_2-$

r) $-(CH_2)_3-O-(CH_2)_{2\text{ bis }4}-O-(CH_2)_3-$

s) 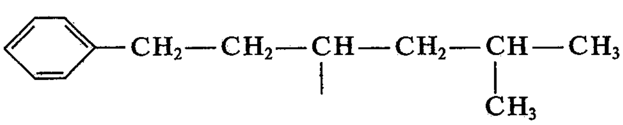

Die für die Herstellung der erfindungsgemäßen Glycidyl-1,2,4-triazolidin-3,5-dione der Formel I notwendigen Ausgangstriazolidin-3,5-dione können nach verschiedenen Verfahren erhalten werden.

So können Amine der Formel II

$$R^1(NH_2)_n \qquad \text{(II)}$$

worin

$R^1$ und n die für Formel I angegebene Bedeutung besitzen,

mit Hydrazodicarbonamid (Verfahren 1) oder mit 1,2,4-Triazolodin-3,5-dion (Verfahren 2) bei 150—280°C in Gegenwart oder Abwesenheit eines Lösungsmittels wie N-Methylpyrrolidon oder Lösungsmittelgemisches bei Drücken von 50 mbar bis 5 bar, gegebenenfalls in Gegenwart eines sauren oder basischen Katalysators wie Alkoholaten oder tert. Aminen, unter Abspaltung von Ammoniak zu den Ausgangs-1,2,4-triazolidin-3,5-dionen umgesetzt werden.

Eine weitere Möglichkeit für die Herstellung der den Ausgangs-1,2,4-triazolidin-3,5-dionen umgesetzt werden.

Eine weitere Möglichkeit für die Herstellung der Ausgangs-1,2,4-triazolidin-3,5-dione besteht darin, daß man N-monosubstituierte Hydrazodicarbonamide der Formel III

$$[H_2N-CO-NH-NH-CO-NH-]_nR^1 \qquad (III)$$

worin

$R^1$ und n die in Formel I angegebene Bedeutung haben,

unter den Bedingungen, wie vorstehend für die Verfahren 1 und 2 angegeben, unter Ammoniakabspaltung zu den Ausgangs-1,2,4-triazolidin-3,5-dionen umsetzt. Die N-monosubstituierten Hydrazodicarbonamide der Formel III können nach bekannten Verfahren aus Semicarbazid und Isocyanaten der Formel IV

$$R^1(NCO)_n \qquad (IV)$$

worin

$R^1$ und n die für Formel I angegebene Bedeutung haben,

erhalten werden.

Die erfindungsgemäßen Glycidyl-1,2,4-triazolidin-3,5-dione der allgemeinen Formel I werden erhalten, indem man 1,2,4-Triazolidin-3,5-dione der Formel V

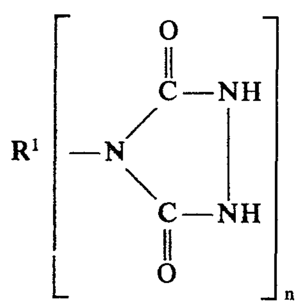

(V)

worin

$R^1$ und n die in Formel I angegebene Bedeutung haben,

mit einem Epihalogenhydrin oder $\beta$-Methylepihalogenhydrin wie z. B. Epichlorhydrin, Epibromhydrin oder $\beta$-Methylepichlorhydrin, gegebenenfalls in Gegenwart eines geeigneten Katalysators, oder mit 1,3-Dihalogenhydrinpropan in Gegenwart Halogenwasserstoff abspaltender Mittel in an sich bekannter Weise umsetzt und anschließend durch Behandlung mit Halogenwasserstoffakzeptoren Halogenwasserstoff abspaltet. Die Herstellung der Glycidyl-triazolidin-3,5-dione der Formel I kann auch einstufig durch Umsetzung der 1,2,4-Triazolidin-3,5-dione der Formel V mit einem Epihalogenhydrin oder 1,3-Dihalogenhydrinpropan in Gegenwart Halogenwasserstoff abspaltender Mittel, wie Natrium- oder Kaliumhydroxid, erfolgen.

Beim bevorzugt verwendeten zweistufigen Verfahren werden in einer ersten Stufe 1,2,4-Triazolidin-3,5-dione der Formel V mit einem Epihalogenhydrin in Anwesenheit von basischen, sauren oder neutralen Katalysatoren zur Halogenhydrinverbindung der allgemeinen Formel VI

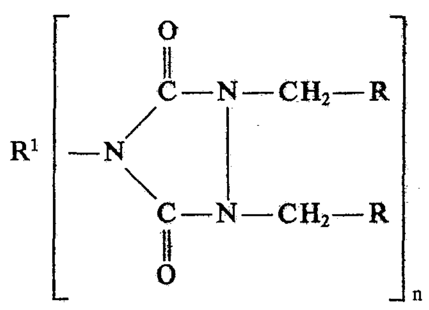

(VI)

in der die Reste R in 1,2-Epoxyethylreste umwandelbare Reste bedeuten, umgesetzt. $R^1$ und n haben die für Formel I angegebene Bedeutung.

Ein solcher, in einen 1,2-Epoxyethylrest umwandelbarer Rest R ist vor allem ein an verschiedenen Kohlenstoffatomen funktionelle Gruppen tragender Hydroxyhalogenethylrest wie die 2-Chlor-1-hydroxy- oder 2-Methyl-2-chlor-1-hydroxyethylreste.

Vorzugsweise verwendet man bei der Addition des Halogenhydrins als Katalysatoren tertiäre Amine und/oder quaternäre Ammoniumsalze wie Trimethylbenzylammoniumhydroxid, Tetraethylammoniumchlorid, Trimethylbenzylammoniumchlorid und Trimethyl-phenylammoniumchlorid, Triethylamin, Tri-n-butylamin, Triethanolamin, N,N'-Dimethylanilin, Benzyldimethylamin, Pyridin, Endoethylenpiperazin und N,N'-Dimethylpiperazin.

Ferner sind auch handelsübliche basische Ionenaustauscherharze mit tertiären oder quaternären Aminogruppen sowie mit Säureamidgruppen geeignet.

Als Katalysatoren sind auch niedermolekulare Thioether und/oder deren Sulfoniumsalze wie Diethylsulfid, Dibenzylsulfid, β-Hydroxyethyl-ethylsulfid, Thiodiglykol und Dibenzyl-methylsulfoniumbromid bzw. Verbindungen, welche mit Epihalogenhydrin in Thioether oder deren Sulfoniumverbindungen übergehen können, wie beispielsweise Schwefelwasserstoff, Natriumsulfid oder Mercaptane zu verwenden.

Es können jedoch auch Alkalimetall- und Erdalkalimetallsalze wie Lithiumchlorid und Calciumrhodanid eingesetzt werden.

Die Menge an Katalysator liegt vorzugsweise zwischen 0,01 und 5 Mol-%, bezogen auf eingesetztes 1,2,4-Triazolidin-3,5-dion der Formel V.

Die Halogenhydrinverbindung der allgemeinen Formel (VI) kann auch hergestellt werden durch die Umsetzung von 1,2,4-Triazolidin-3,5-dion der Formel V mit Dihalogenhydrinpropan wie 1,3-Dichlorpropanol-2 oder 2-Methyl-1,3-dichlorpropanol-2 in Gegenwart von Halogenwasserstoff abspaltenden Mittel wie Alkali- oder Erdalkalihydroxide, z. B. Natrium- und Bariumhydroxid, Alkali- oder Erdalkalicarbonate, z. B. Soda und Calciumcarbonat und tertiäre Amine wie Triethylamin.

Die Reaktion der 1,2,4-Triazolidin-3,5-dione der Formel V mit dem Epihalogenhydrin wird mit mindestens äquivalenten Mengen an Epihalogenhydrin durchgeführt, d. h. eine NH-Gruppe der 1,2,4-Triazolidin-3,5-dione der Formel V wird mit mindestens einem Mol Epihalogenhydrin zur Reaktion gebracht. Vorzugsweise wird jedoch ein Überschuß an Epihalogenhydrin, und zwar 1,2 bis 20 Mol, besonders bevorzugt 3 bis 10 Mol Epihalogenhydrin pro NH-Gruppe, verwendet. Aus wirtschaftlichen Gründen wird man die Menge an Epihalogenhydrin natürlich so niedrig wie möglich halten.

Die Reaktion zwischen den 1,2,4-Triazolidin-3,5-dionen der Formel V und Epihalogenhydrin wird bei 20 bis 200° C, vorzugsweise bei 50 bis 160° C, gegebenenfalls unter erhöhtem Druck, durchgeführt.

Die Reaktionszeiten liegen im allgemeinen zwischen 30 Minuten und mehreren Tagen, können in besonderen Fällen jedoch auch darüber oder darunter liegen. Durch entsprechende Wahl der Reaktionsbedingungen, z. B. Druck, werden kürzere Reaktionszeiten erreicht.

Prinzipiell kann die Umsetzung der 1,2,4-Triazolidin-3,5-dione der Formel V mit dem Epihalogenhydrin oder β-Methylhalogenhydrin auch ohne Zusatz von Katalysatoren durchgeführt werden, jedoch sind dann höhere Temperaturen und/oder längere Reaktionszeiten als vorstehend angegeben, erforderlich.

Anschließend wird in einer zweiten Stufe die Halogenhydrinverbindung, die jedoch je nach Epihalogenhydrin- oder β-Methylhalogenhydrinüberschuß bereits gewisse Mengen an Glycidylverbindungen enthalten kann, mittels Halogenwasserstoff-abspaltender Verbindungen zu Glycidyl-1,2,4-triazolidin-3,5-dionen der Formel I dehydrohalogeniert.

Die zur Halogenwasserstoffabspaltung eingesetzten alkalisch reagierenden Verbindungen sind insbesondere Alkali- oder Erdalkalimetallhydroxide wie Natriumhydroxid, Kaliumhydroxid, Calciumhydroxid, vorzugsweise Natriumhydroxid. Dies kann in fester Form oder in Lösung, vorzugsweise in 20—50%igen Lösungen, eingesetzt werden.

Ferner können zur Halogenwasserstoffabspaltung Alkalicarbonate, insbesondere Soda und Kaliumcarbonat in fester oder gelöster Form, Alkalisilikate, Alkaliphosphate und Alkalialuminate sowie überschüssiges Epihalogenhydrin, oder 1,2-Alkylenoxide, wie Ethylenoxid Verwendung finden, wobei bei Verwendung von Epichlorhydrin dieses in Glycerindichlorhydrin umgewandelt wird.

Auf eine Halogenhydrin-Gruppe der 1,2,4-Triazolidin-3,5-dione der Formel VI werden 1 bis 1,2 Äquivalente der Halogenwasserstoff abspaltenden Verbindungen eingesetzt.

Zweckmäßig soll bei der Halogenwasserstoffabspaltung der pH-Wert nicht über 13, vorzugsweise nicht über pH 11 ansteigen. Um dies zu erreichen, setzt man das Alkali nach und nach zu bzw. tropft die Lösung allmählich zu und kontrolliert dabei den pH-Wert der Reaktionsmischung.

Die Halogenwasserstoffabspaltung kann im Temperaturbereich von 5°C bis 120°C erfolgen. Wird die Halogenwasserstoffabspaltung mit Alkali, z. B. Kalilauge, durchgeführt, so ist zur Erzielung optimaler Ausbeuten eine Reaktionstemperatur von 70°C nicht zu überschreiten. Die besten Ergebnisse werden bei Temperaturen um 30 bis 35°C erhalten. Bei Einsatz von Alkalicarbonaten sollte die Reaktionstemperatur jedoch über 70°C liegen, wobei die Höchstgrenze in der Regel durch die Siedetemperatur des überschüssigen Epihalogenhydrins vorgegeben ist.

Bei der Dehydrohalogenierung ist es vorteilhaft, ein mit Wasser nicht mischbares organisches Lösungsmittel zuzusetzen, um das bei der Reaktion entstehende bzw. das über die Alkalilösung zugetropfte Wasser azeotrop entfernen zu können. Die Mengen an zugesetztem Lösungsmittel sind nicht kritisch. Geeignete Lösungsmittel sind chlorierte Kohlenwasserstoffe wie Methylenchlorid, Chloroform, Ethylenchlorid oder Trichlorethylen. Sofern für die Bildung der 1,2,4-Triazolidin-3,5-dion-chlorhydrinverbindungen der Formel VI ein großer Überschuß an Epihalogenhydrin eingesetzt wurde, kann das überschüssige Epihalogenhydrin als mit Wasser nicht mischbares Lösungsmittel fungieren.

Eine besonders bevorzugte Ausführungsform besteht darin, daß man 1,2,4-Triazolidin-3,5-dione der Formel V mit Epihalogenhydrin, vorzugsweise Epichlorhydrin, in Gegenwart eines Katalysators, vorzugsweise eines tert. Amins, einer quaternären Ammoniumbase, eines quaternären Ammoniumsalzes oder eines organischen Sulfids bzw. Sulfoniumsalzes, in einer ersten Stufe umsetzt und das dabei entstandene Halogenhydringruppen enthaltende 1,2,4-Triazolidin-3,5-dion in einer zweiten Stufe mit halogenwasserstoffabspaltenden Verbindungen, insbesondere Alkali-, Erdalkalihydroxiden oder Alkalicarbonaten in fester oder gelöster Form, behandelt.

Die Aufarbeitung der 1,2,4-Glycidyl-triazolidin-3,5-dione der Formel I erfolgt in der Regel so, daß das bei der Halogenwasserstoffabspaltung entstehende Nebenprodukt, z. B. Kochsalz bei Verwendung von Natronlauge als Säurefänger, durch Absaugen entfernt wird. Eventuell noch vorhandene Kochsalz- und Alkalireste werden durch Auswaschen mit Wasser entfernt. Selbstverständlich können jedoch auch das gesamte Kochsalz und eventuell vorhandene Alkalireste ohne vorheriges Absaugen durch Auswaschen mit Wasser entfernt werden. Die verbleibende Lösung wird dann gegebenenfalls nach dem Trocknen über einem geeigneten Trocknungsmittel wie wasserfreiem Natriumsulfat, vom Lösungsmittel, z. B. überschüssigem Epichlorhydrin, welches in den nächsten Ansätzen wieder mitverwendet werden kann, gegebenenfalls im Vakuum entfernt, und das erhaltene hellgelb bis gelb gefärbte viskose Öl wird gegebenenfalls durch Auflösen in geeigneten Lösungsmitteln wie $C_1-C_4$-Alkanolen, vorzugsweise Methanol, Ketonen wie Butanon, Glykol- bzw. Diglykolmonoether oder deren Acetate wie Ethylen-glykolmonomethylether, Diethylen-glykolmonoethylether und Ethylenglykolmonomethyletheracetat und nachfolgendem Abkühlen zur Kristallisation gebracht.

Die erhaltene, kristallinen Verbindungen, können abgesaugt und gegebenenfalls durch Umkristallisieren, z. B. aus Methanol, nochmals gereinigt werden. Häufig kann jedoch auf eine Reinigung verzichtet und das Rohprodukt sofort weiterverarbeitet werden. Je nach Konstitution des Restes $R^1$ entstehen häufig jedoch dünnflüssig bis hochviskose Harze, die dann ohne weitere Reinigung weiter umgesetzt werden können.

Die erfindungsgemäßen 1,2,4-Glycidyl-triazolidin-3,5-dione der Formel I besitzen Epoxidwerte von 0,2 bis 0,91, vorzugsweise 0,64 bis 0,91. Unter Epoxidwerte wird die Anzahl von Epoxidäquivalenten verstanden, die in 100 g Substanz enthalten ist. Das Epoxidäquivalent ist definiert als die Grammenge Substanz, in der eine 1,2-Epoxidgruppe enthalten ist. Eine 1,2-Epoxidgruppe ist einem Mol Halogenwasserstoff äquivalent. Von der Herstellung her können die Polyglycidylverbindungen der 1,2,3-Triazolidin-3,5-dione noch verseifbares Halogen (bis ca. 5 Gew.-% Chlor bzw. ca. 13 Gew.-% Brom) enthalten, das durch weitere Behandlung mit Halogenwasserstoff abspaltenden Mitteln, falls gewünscht, praktisch vollständig entfernt werden kann und zumindest teilweise den Gehalt an Epoxidgruppen erhöht.

Prinzipiell ist es möglich, die erfindungsgemäßen Verbindungen der Formel (I) aus Verbindungen der Formel (VI), in der R einen Ethenylrest ($CH_2=CH-$) darstellt, durch Peroxidierung, z. B. mittels Wasserstoffperoxid oder Persäuren herzustellen.

Die erfindungsgemäßen 1,2,4-Glycidyl-triazolidin-3,5-dione der Formel I, Polyglycidylverbindungen können, allein oder zusammen mit üblichen Härtungsmitteln, als Imprägnierungsmittel für Textilien, z. B. Fasern aus Polyester, als Überzugsmittel, z. B. für Anstrichzwecke auf Glas, Metallen und Holz, als Klebemittel für Kunststoffe verschiedenster Art, z. B. zum Verkleben von ungewebten textilartigen Gebilden, sowie zur Herstellung von Formkörpern, z. B. Gieß-, Preßkörpern und Laminaten verwendet werden.

Die nachfolgenden Beispiele sollen die Erfindung näher erläutern, wobei, wenn nicht anders angegeben, die Prozente und Teile sich auf das Gewicht beziehen.

## Beispiele

### Herstellung der Ausgangs-1,2,4-triazolidin-3,5-dione der Formel V

#### Ausgangsstoff 1

600 g Hydrazodicarbonamid und 150 g Ethylendiamin werden in 500 ml N-Methylpyrrolidon 4 Stunden bei 175°C und 20 Stunden bei 200°C gerührt. Beim Abkühlen fällt ein Niederschlag aus, der abgesaugt und mit Ethanol gewaschen wird. Man erhält 462 g (80% der Theorie) 1,2-Ethandiyl-4,4'-bis-1,2,4-triazolidin-3,5-dion als farblose Kristalle vom Fp. >330°C.

| | | | | |
|---|---|---|---|---|
| $C_6H_8N_6O_4$ | ber.: | C 31,58 | H 3,53 | N 36,83 |
| (228,2) | gef.: | C 31,4 | H 3,6 | N 36,8 |

IR(KBr): 1731, 1673 $cm^{-1}$ (C=O).

#### Ausgangsstoff 2

420 g 4,4'-Diaminodicyclohexylmethan und 472 g Hydrazodicarbonamid werden in 750 ml N-Methylpyrrolidon 4 Stunden bei 175°C, 8 Stunden bei 200°C und 4 Stunden bei 220°C gerührt. Das Reaktionsprodukt wird abgekühlt und anschließend in 3 l Wasser eingerührt. Dabei fällt ein Niederschlag aus, der abgesaugt und mit Wasser gewaschen wird. Man erhält 566 g (75% der Theorie) an 4,4'-Bis-(1,2,4-triazolidin-3,5-dion-4-yl)-dicyclohexylmethan als farblose Kristalle vom Fp. 305°C (Zersetzung).

MS (m/e): Molpeak 378.

#### Ausgangsstoff 3

Zu einer Lösung von 111,5 g Semicarbazidhydrochlorid in 700 g Wasser wird so lange in kleinen Portionen Soda zugesetzt, bis keine Gasentwicklung mehr zu beobachten ist.

Dann wird bei 40°C eine Lösung aus 119 g Phenylisocyanat in 100 g Aceton zugetropft. Zur Vervollständigung der Reaktion wird 2 Stunden bei 40°C nachgerührt und der gebildete Niederschlag durch Absaugen isoliert.

Der über Nacht an der Luft getrocknete Niederschlag wird in 300 g Sulfolan suspendiert und auf 205°C aufgeheizt, wobei ab 160°C durch Anlegen eines Wasserstrahlvakuums von 420 mbar das freiwerdende Ammoniak abgezogen wird. Nach einer Reaktionszeit von 5 Stunden wird bei einem Druck von 0,3 mbar das Lösungsmittel größtenteils entfernt und der verbliebene Rückstand aus n-Butanol umkristallisiert. Nach dem Absaugen und Trocknen werden 134 g 4-Phenyl-1,2,4-triazolidin-3,5-dion vom Fp. = 202—203°C (Lit. 203°C) erhalten.

#### Ausgangsstoffe 4

60 g Hydrazodicarbonamid und 29 g 1,6-Diaminohexan werden in 100 ml Sulfolan 2 Stunden bei 175°C und 9 Stunden bei 200°C gerührt. Beim Abkühlen fällt ein Niederschlag aus, der abgesaugt und aus Wasser umkristallisiert wird. Man erhält 36 g (51% d. Th.) 1,6-Hexandiyl-4,4'-bis-1,2,4-triazolidin-3,5-dion als farblose Kristalle vom Fp.: 215—217°C.

| | | | | |
|---|---|---|---|---|
| $C_{10}H_{16}N_6O_4$ | ber. | C 42,25 | H 5,67 | N 29,51 |
| (284,3) | gef. | C 42,5 | H 5,7 | N 29,2 |

#### Ausgangsstoff 5

102 g 1,4-Butandiolbis-(3-aminopropylether) und 120 g Hydrazodicarbonamid werden in 300 ml N-Methylpyrrolidon 1 Stunde bei 150°C, 2 Stunden bei 175°C und 5 Stunden bei 200°C gerührt. Beim Abkühlen fällt ein Niederschlag aus, der abgesaugt und mit 200 ml Acetonitril ausgekocht wird. Als Rückstand erhält man 142 g (76% d. Th.) 1,4-Butandiol-bis-[3-(3,5-dioxo-1,2,4-triazolidin-4-yl)-propylether] als farblose Kristalle vom Fp.: 152—154°C.

| | | | | |
|---|---|---|---|---|
| $C_{14}H_{24}N_6O_6$ | ber. | C 45,15% | H 6,50% | N 22,57% |
| (372,4) | gef. | C 45,2% | H 6,6% | N 22,6% |

IR (KBr): 1768, 1674 $cm^{-1}$ (C=O).

Ausgangsstoff 6

111,5 g Semicarbazidhydrochlorid werden in 400 ml $H_2O$ gelöst und mit Soda neutralisiert. Anschließend werden unter starkem Rühren bei Raumtemperatur 57 g Methylisocyanat, gelöst in 300 ml Dioxan innerhalb von 1 Stunde zugetropft. Man rührt 2 Stunden bei Raumtemperatur nach und saugt vom entstandenen Niederschlag ab. Der Niederschlag wird in 1 l N-Methylpyrrolidon suspendiert und 10 Stunden bei 200°C und 300 mbar pyrrolysiert. Anschließend wird das Lösungsmittel im Vakuum abdestilliert und der Rückstand aus Ethanol umkristallisiert. Man erhält 96 g (83% d. Th.) 4-Methyl-1,2,4-triazolidin-3,5-dion als farblose Kristalle vom Fp.: 230—232°C. (Lit. 232—233°C).

Ausgangsstoff 7

101 g 1,2,4-Triazolidin-3,5-dion und 57 g 1,4-Diaminocyclohexan werden in 500 ml N-Methylpyrrolidon 4 h auf 175°C und 30 Stunden auf 200°C unter Rühren erhitzt. Beim Abkühlen kristallisiert ein Niederschlag aus, der abgesaugt, mit Ethanol gewaschen und getrocknet wird. Man erhält 110 g (78% d. Th.) 1,4-Cyclohexandiyl-bis-(1,2,4-triazolidin-3,5-dion-4-yl) als farblose Kristalle vom Fp. >300°C.

| | | | | |
|---|---|---|---|---|
| $C_{10}H_{14}N_6O_4$ | ber. | C 42,56% | H 4,96% | N 29,75% |
| (282,2) | gef. | C 42,7% | H 5,1% | N 29,1% |

MS (m/e): Molpeak 282

Ausgangsstoff 8

100 g 1,12-Diaminododecan und 120 g Hydrazodicarbonamid werden in 300 ml N-Methylpyrrolidon 1 Stunde bei 175°C und 4 Stunden bei 200°C gerührt. Beim Abkühlen fällt ein Niederschlag aus, der abgesaugt und mit 300 ml Acetonitril ausgekocht wird. Man erhält 156 g (85% d. Th.) 1,2-Dodecan-4,4'-bis-(1,2,4-triazolidin-3,5-dion) als farblose Kristalle von Fp. 172—175°C.

| | | | | |
|---|---|---|---|---|
| $C_{16}H_{28}N_6O_4$ | ber. | C 52,16% | H 7,66% | N 22,81% |
| (368,5) | gef. | C 52,2% | H 7,7% | N 22,6% |

MS (m/e): Molpeak 368.

Beispiel 1

2000 g Epichlorhydrin, 228 g 1,2-Ethandiyl-4,4'-bis-(1,2,4-triazolidin-3,5-dion) (Ausgangsstoff 1), 2 g Triethylamin und 200 ml Wasser werden 3,5 Stunden unter Rückfluß gerührt. Anschließend wird das Wasser durch azeotrope Destillation am Wasserabscheider entfernt. Zum Reaktionsgemisch werden bei 40—45°C unter vermindertem Druck 170 g Natriumhydroxid als 45%ige wäßrige Lösung zugetropft und gleichzeitig Wasser durch azeotrope Destillation am Wasserabscheider entfernt. Anschließend wird so lange bei 40—45°C nachgerührt, bis alles Wasser entfernt ist. Man saugt vom entstandenen Natriumchlorid ab und wäscht es zweimal mit je 200 g Epichlorhydrin. Die vereinigten Epichlorhydrinlösungen werden mit 200 ml Wasser ausgeschüttet. Nach dem Trocknen der organischen Phase über Natriumsulfat wird das Lösungsmittel im Vakuum entfernt und der Rückstand zuletzt bei 60°C und 0,3 mbar bis zur Gewichtskonstanz getrocknet. Es werden 421 g eines hellgelben hochviskosen Harzes erhalten, dessen Epoxidwert 0,82 und dessen Chlorgehalt = 1,35% Gesamtchlor beträgt.

Beispiel 2

37,8 g 4,4'-Bis-(1,2,4-triazolodin-3,5-dion-4-yl)-di-cyclohexylmethan (Ausgangsstoff 2), 0,1 g Triethylamin und 300 g Epichlorhydrin werden 5 Stunden unter Rückfluß gerührt. Zu der auf 30°C abgekühlten Lösung werden innerhalb von 2 Stunden in kleinen Portionen 16,5 g pulverisiertes Ätznatron zugesetzt und zur Vervollständigung der Reaktion noch 2 Stunden bei 30°C nachgerührt. Das entstandene Kochsalz wird durch Filtration abgetrennt und zweimal mit je 30 g Epichlorhydrin gewaschen. Die vereinigten Epichlorhydrinlösungen werden mit 50 ml Wasser ausgeschüttelt. Nach dem Trocknen der

organischen Phase über Natriumsulfat wird das Lösungsmittel im Vakuum entfernt und der Rückstand zuletzt bei 60° C und 0,3 mbar bis zur Gewichtskonstanz getrocknet. Es werden 52,1 g eines hellgelben hochviskosen Öles erhalten, dessen Epoxidwert 0,59 und dessen = 2,1% Gesamtchlor beträgt.

Beispiel 3

70,8 g 4-Phenyl-1,2,4-triazolidin-3,5-dion (Ausgangsstoff 3), 1 g Tetraethylammoniumchlorid und 500 g Epichlorhydrin werden 5 Stunden bei 80° C gerührt. Nach dem Abkühlen auf 40° C werden innerhalb von 2 Stunden 85 g 40%ige Natronlauge zugetropft, wobei das eingebrachte und das bei der Reaktion freiwerdende Wasser über einen Wasserabscheider bei vermindertem Druck aus dem Reaktionsgemisch entfernt wird. Zur Vervollständigung der Reaktion wird noch eine Stunde bei 40° C unter azeotropen Rückfluß nachgerührt. Das entstandene Kochsalz wird durch Filtration abgetrennt und mit 100 g Epichlorhydrin gewaschen. Die vereinigten Epichlorhydrinlösungen werden zweimal mit 30 ml Wasser gewaschen und über Natriumsulfat getrocknet. Anschließend wird das Epichlorhydrin im Vakuum abdestilliert. Der bei 70° C und 0,2 mbar bis zur Gewichtskonstanz getrocknete Rückstand wiegt 107 g. Er hat einen Epoxidwert von 0,66 und einen Chlorgehalt von 1,3% Gesamtchlor.

Beispiel 4

56,8 g 1,6-Hexandiyl-4,4′-bis-(1,2,4-triazolidin-3,5-dion) (Ausgangsstoff 4), 0,5 g Thioglykol und 1,2 kg Epichlorhydrin werden 7 Stunden bei 100° C gerührt. Nach dem Abkühlen werden bei 50° C 46 g Kaliumhydroxid als 25%ige wäßrige Lösung innerhalb von 1,5 Stunden zugetropft, wobei das eingebrachte und das bei der Reaktion entstehende Wasser über einen Wasserabscheider bei vermindertem Druck aus dem Reaktionsgemisch entfernt wird. Zur Vervollständigung der Reaktion wird noch 2 Stunden bei 50° C unter azeotropem Rückfluß nachgerührt. Das entstandene Salz wird durch Auswaschen mit Wasser entfernt. Die Epichlorhydrinlösung wird anschließend über Natriumsulfat getrocknet und im Vakuum eingeengt. Nach dem Trocknen bei 60° C und 0,25 mbar bis zur Gewichtskonstanz werden 95 g eines viskosen, gelblich gefärbten Epoxidharzes mit einem Epoxidwert von 0,73 und einem Chlorgehalt von 1,6% Gesamtchlor erhalten.

Beispiel 5

55,8 g 1,4-Butanbisoxypropyl-3-diyl-[1,2,4-Triazolidin-3,5-dion-4-yl] (Ausgangsstoff 5), 0,2 g Triethylamin und 1,2 kg Epichlorhydrin werden 10 Stunden bei 80° C gerührt. Nach dem Abkühlen werden 26 g pulverisiertes Natriumhydroxid portionsweise innerhalb 3 Stunden bei 30° C zugegeben. Das bei der Reaktion entstandene Wasser wird als Azeotrop mit Epichlorhydrin abdestilliert. Durch Filtrieren wird das Kochsalz entfernt und das restliche Epichlorhydrin im Vakuum abdestilliert. Nach dem Trocknen bei 70° C und 0,4 mbar zur Gewichtskonstanz wird ein gelblich gefärbtes Öl mit einem Epoxidwert von 0,61 und einem Chlorgehalt von 0,7% Gesamtchlor erhalten.

Beispiel 6

a) 115 4-Methyl-1,2,4-triazolidin-3,5-dion (Ausgangsstoff 6), 1 g Triethylamin und 750 g Epichlorhydrin werden 4 Stunden bei Rückfluß gerührt. Nach dem Abkühlen werden bei 35° C 82 g Natriumhydroxid, gelöst in 100 g Wasser innerhalb von 4 Stunden zugetropft, wobei das Wasser über einen Wasserabscheider bei vermindertem Druck aus dem Reaktionskolben entfernt wird. Zur Vervollständigung der Reaktion wird noch 3 Stunden bei 35° C und azeotropem Rückfluß bei vermindertem Druck nachgerührt, unlösliche Bestandteile durch Absaugen entfernt und die Epoxidharzlösung durch Waschen mit Wasser von anorganischen Bestandteilen befreit. Nach dem Trocknen der Epoxidharzlösung über $Na_2SO_4$ wird am Rotationsverdampfer eingeengt und der Rückstand bei 60° C und 0,25 mbar bis zur Gewichtskonstanz getrocknet. Es verbleiben 190 g eines viskosen, gelb gefärbten Epoxidharzes mit einem Epoxidwert von 0,79 und einem Chlorgehalt von 1,1% Gesamtchlor, welches nach zweitägigem Stehen erstarrt. Durch Verreiben mit Ethanol wird praktisch reines 1,2-Bisglycidyl-4-methyl-1,2,4-triazolidin-3,5-dion vom Fp. = 115—116° C (aus Ethanol) erhalten.

IR- und NMR-Spektren sowie die Elementaranalyse bestätigen die angenommene Struktur

| | | | | |
|---|---|---|---|---|
| $C_9H_{13}N_3O_4$ | C 47,57% | H 5,77% | N 18,50% | Epoxidwert 0,88 |
| (227,2) | C 47,6% | H 5,7% | N 18,6% | Epoxidwert 0,88 |

b) Zu 100 g eines zuvor entgasten, rohen Epoxidharzes mit einem Epoxidwert von 0,79 (aus Beispiel 6a) werden unter Kühlen 21 g Triethylentetramin eingerührt und entgast. Die Masse wird in eine Form gegossen und bei Raumtemperatur gehärtet. Nach 12 Tagen erhält man einen harten Formling von hellgelber Farbe.

c) 100 g kristallines 1,2-Bisglycidyl-4-methyltriazolidin-3,5-dion und 135 g Hexahydrophthalsäureanhydrid werden getrennt aufgeschmolzen, bei 120°C vermischt und in eine Form gegossen. Nach der Härtung von 10 Stunden bei 120°C und 2 Stunden bei 160°C werden Prüfkörper mit einem Martensgrad von 142°C erhalten.

Beispiel 7

141 g 1,4-Cyclohexandiyl-bis-(1,2,4-triazolidin-3,5-dion-4-yl) (Ausgangsstoff 7), 0,5 ml Triethylamin und 1 kg Epichlorhydrin werden 15 Stunden bei 100°C gerührt. Nach dem Abkühlen werden bei 35°C 94 g einer 50%igen Natronlauge innerhalb von 4 Stunden zugetropft, wobei das Wasser über einen Wasserabscheider bei vermindertem Druck aus dem Reaktionsgefäß entfernt wird. Zur Vervollständigung der Reaktion wird noch 3 Stunden bei 35°C und azeotropem Rückfluß bei vermindertem Druck nachgerührt. Die Epoxidharzlösung wird durch Absaugen von unlöslichen Bestandteilen und durch Auswaschen mit Wasser von anorganischen Bestandteilen befreit. Die über Natriumsulfat getrocknete Epoxidlösung wird im Vakuum, zuletzt bei 60°C und 0,3 mbar, eingeengt. Es werden 187 g eines viskosen Epoxidharzes mit einem Epoxidwert von 0,71 und einem Chlorgehalt von 1,1% Gesamtchlor erhalten.

Beispiel 8

184 g 1,12-Dodecandiyl-4,4'-bis-(1,2,4-triazolidin-3,5-dion) (Ausgangsstoff 8), 0,5 ml Triethylamin und 1 kg Epichlorhydrin werden 10 Stunden bei 80°C gerührt. Nach dem Abkühlen auf 35°C wird die Reaktion analog Beispiel 7 weitergeführt. Es werden 271 g eines viskosen Epoxidharzes mit einem Epoxidwert von 0,52 und einem Chlorgehalt von 3,9% Gesamtchlor erhalten.

Beispiel 9

200 g des nach Beispiel 8 erhaltenen Epoxidharzes werden in 500 g Methylenchlorid gelöst. Zu dieser Lösung wird bei Rückfluß des Methylenchlorids innerhalb von 2 Stunden eine dem Chlorgehalt äquivalente Menge (+10% Überschuß) an Natriumhydroxid als 45%ige Natronlauge zugetropft, wobei das Wasser über einen Wasserabscheider aus dem Reaktionsgefäß entfernt wird. Nach Beendigung der Reaktion wird abgesaugt, die organische Phase mit Wasser gewaschen und eingeengt, zuletzt bei 60°C/0,3 mbar. Es entstehen 188 g eines hellgelben Epoxidharzes mit einem Epoxidwert von 0,56 und einem Chlorgehalt von 0,4% Gesamtchlor.

**Patentansprüche**

1. Glycidyl-1,2,4-triazolidin-3,5-dione der allgemeinen Formel I

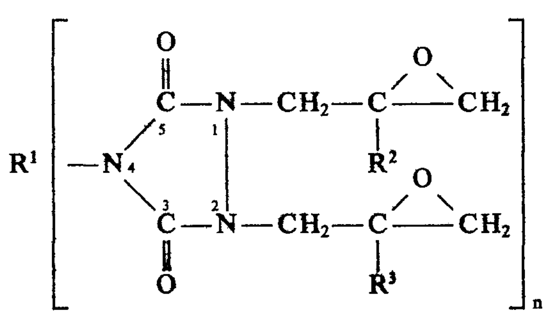

(I)

worin

$R^1$ einen ein- bis fünfwertigen linearen oder verzweigten aliphatischen $C_1-C_{30}$, einen ein- bis fünfwertigen cycloaliphatischen $C_5-C_{21}$, einen ein- bis fünfwertigen aliphatisch-aromatischen $C_7-C_{17}$, oder einen ein- bis fünfwertigen aromatischen $C_6-C_{21}$ Rest, die vorgenannten Reste können durch mindestens eine Alkoxycarbonylgruppe mit $C_1-C_4$ in der Alkoxygruppe, CN, $NO_2$, Alkylmercaptogruppe mit $C_1-C_4$ in der Alkylgruppe, Dialkylaminogruppe mit $C_1-C_6$ in jeder Alkylgruppe, Halogen, und im Falle der aromatischen Reste außer durch die vorgenannten Substituenten durch mindestens eine $C_1-C_4$-Alkylgruppe

substituiert sein, ferner können die vorgenannten aliphatischen Reste durch ein oder mehrere Sauerstoffatome oder tertiäre Stickstoffatome, die vorgenannten mehrkernigen cycloaliphatischen, mehrkernigen aliphatisch-aromatischen und mehrkernigen aromatischen Reste durch mindestens ein Sauerstoff- oder tertiäres Stickstoffatom oder durch mindestens eine Alkylengruppe mit 1—4 C-Atomen oder durch mindestens eine Sulfonylgruppe

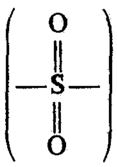

unterbrochen sein,

$R^2$ und $R^3$ gleich oder verschieden für ein Wasserstoffatom oder eine Methylgruppe und

n eine Zahl von 1 bis 5 bedeuten.

2. Glycidyl-1,2,4-triazolidin-3,5-dione gemäß Anspruch 1, dadurch gekennzeichnet, daß $R^2$ und $R^3$ Wasserstoff bedeuten und $R^1$ unsubstituiert ist.

3. Glycidyl-1,2,4-triazolidin-3,5-dione gemäß Anspruch 1, dadurch gekennzeichnet, daß $R^2$ und $R^3$ Wasserstoff und $R^1$ einen der Reste

a) $C_nH_{2n+1}—$ n = 1 bis 18

b) $—(CH_2)_m—$ m = 2 bis 12

c) 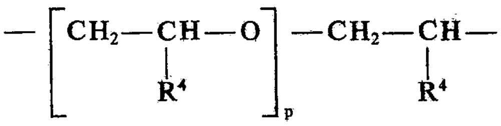 $R^4$ = H, $CH_3$; p = 1–9

d) 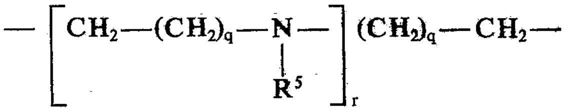 $R^5$ = $C_1$–$C_4$-Alkyl; q = 1–2; r = 1–4

e) 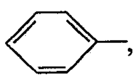, 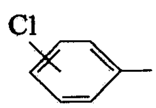

f) 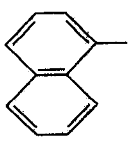

g) 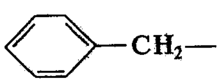

h) 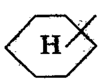

i) 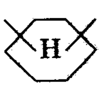

j) 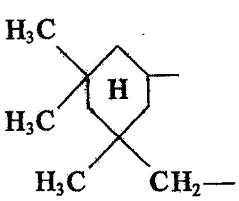

k) 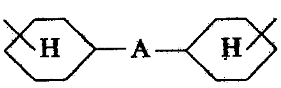 A = Alkylen mit 1–4 C-Atomen, O, $-N(CH_3)-$;

l) 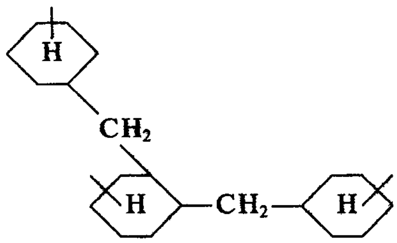

m) 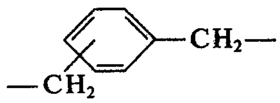

n) 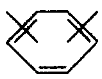

o) 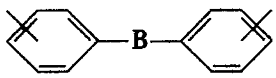

B = Alkylen mit 1–4 C-Atomen, O, $-N(CH_3)-$,

p) 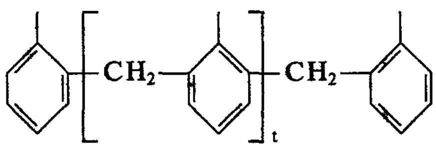

t = 1 bis 3

q) $C_4H_9-O-CH_2-CH_2-CH_2-$

r) $-(CH_2)_3-O-(CH_2)_{2\ \text{bis}\ 4}-O-(CH_2)_3-$

s) 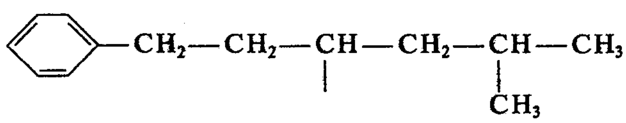

bedeutet.

4. Glycidyl-1,2,4-triazolidin-3,5-dione gemäß Anspruch 1, dadurch gekennzeichnet, daß der Epoxidwert 0,2 bis 0,91 beträgt.

5. Verfahren zur Herstellung von 1,2,4-Glycidyltriazolidin-3,5-dionen gemäß Anspruch 1, dadurch gekennzeichnet, daß 1,2,4-Triazolidin-3,5-dion der Formel V

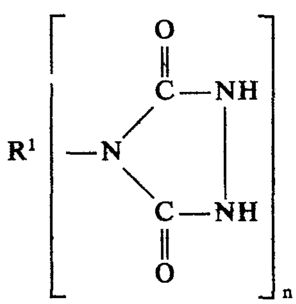 (V)

worin

$R^1$ und n die in Formel I angegebene Bedeutung haben,

mit mindestens der äquivalenten Menge an Epihalogenhydrin oder $\beta$-Methylepihalogenhydrin, gegebenenfalls in Gegenwart eines Katalysators, zu den entsprechenden Poly-(halogenhydrinen) des 1,2,4-Triazolidin-3,5-dions der oben angegebenen Formel umgesetzt werden und anschließend mit Halogenwasserstoff abspaltenden Mittel in 1,2,4-Glycidyl-triazolidin-3,5-dione der Formel I überführt werden.

6. Verfahren zur Herstellung von Glycidyl-1,2,4-triazolidin-3,5-dionen gemäß Anspruch 5, dadurch gekennzeichnet, daß als Epihalogenhydrin Epichlorhydrin verwendet wird.

7. Verfahren gemäß Anspruch 5, dadurch gekennzeichnet, daß pro NH-Gruppe der 1,2,4-Triazolidin-3,5-dione der Formel V 1,2 bis 20 Mol Epichlorhydrin eingesetzt werden.

**Claims**

1. Glycidyl-1,2,4-triazolidine-3,5-diones of the general formula I

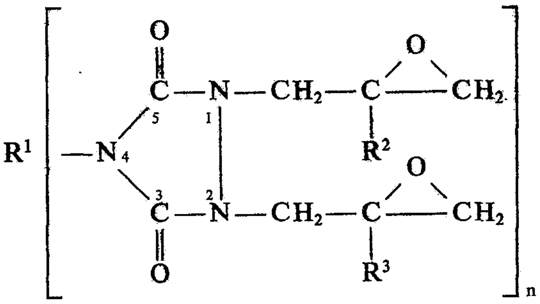

(I)

wherein

$R^1$ denotes a mono- to pentavalent linear or branched aliphatic $C_1-C_{30}$ radical, a mono- to pentavalent cycloaliphatic $C_5-C_{21}$ radical, a mono- to pentavalent aliphatic-aromatic $C_7-C_{17}$ radical or a mono- to pentavalent aromatic $C_6-C_{21}$ radical, the aforementioned radicals can be substituted by at least one alkoxycarbonyl group with $C_1-C_4$ in the alkoxy group, CN, $NO_2$, alkylmercapto group with $C_1-C_4$ in the alkyl group, dialkylamino group with $C_1-C_6$ in each alkyl group, halogen and in the case of the aromatic radicals by at least one $C_1-C_4$-alkyl group in addition to the aformentioned substituents, furthermore the aforementioned aliphatic radicals can be interrupted by one or more oxygen atoms or tertiary nitrogen atoms, the aforementioned polynuclear cycloaliphatic, polynuclear aliphatic-aromatic and polynuclear aromatic radicals can be interrupted by at least one oxygen or tertiary nitrogen atom or by at least one alkylene group with 1—4 C atoms or by at least one sulphonyl group

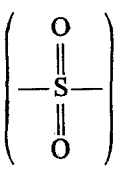

$R^2$ and $R^3$ are identical or different and denote a hydrogen atom or a methyl group and
n denotes a number from 1 to 5.

2. Glycidyl-1,2,4-triazolidine-3,5-diones according to Claim 1, characterised in that $R^2$ and $R^3$ denote hydrogen and $R^1$ is unsubstituted.

3. Glycidyl-1,2,4-triazolidine-3,5-diones according to Claim 1, characterised in that $R^2$ in that $R^2$ and $R^3$ denote hydrogen and $R^1$ denotes one of the radicals

a) $C_nH_{2n+1}-$ n = 1 to 18

b) $-(CH_2)_m-$ m = 2 to 12

c) 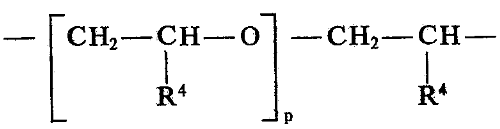 $R^4$ = H, $CH_3$; p = 1–9

d) 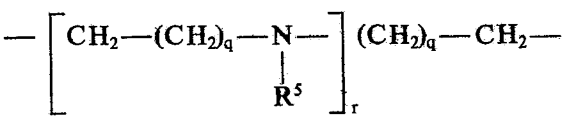 $R^5$ = $C_1-C_4$-alkyl; q = 1–2; r = 1–4

e) 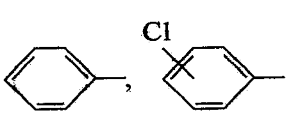

f) 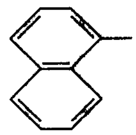

g) 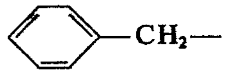

h) 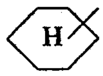

i) 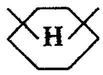

j) 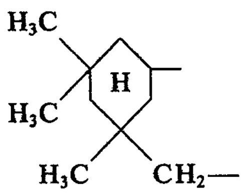

k) 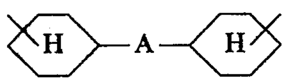

A = alkylene with 1–4 C-atoms, O, $-N(CH_3)-$;

l) 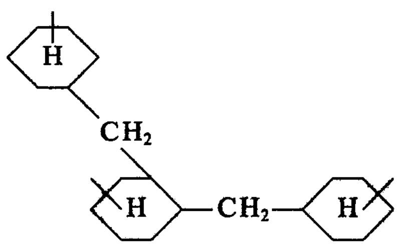

m) 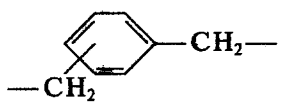

n) 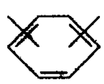

o) 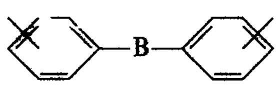

B = alkylene with 1–4 C-atoms, O, $-N(CH_3)-$,

p) 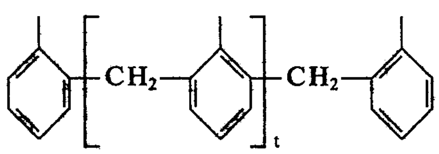

t = 1 to 3

q) $C_4H_9-O-CH_2-CH_2-CH_2-$

r) $-(CH_2)_3-O-(CH_2)_{2\ \text{bis}\ 4}-O-(CH_2)_3-$

s) 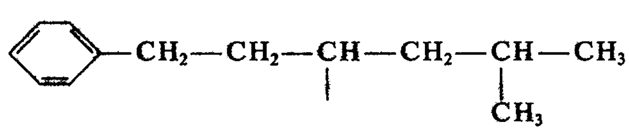

4. Glycidyl-1,2,4-triazolidine-3,5-diones according to Claim 1, characterised in that the epoxide value is 0.2 to 0.91.

5. Process for the preparation of 1,2,4-glycidyl-triazolidine-3,5-diones according to Claim 1, characterised in that 1,2,4-triazolidine-3,5-dione of the formula V

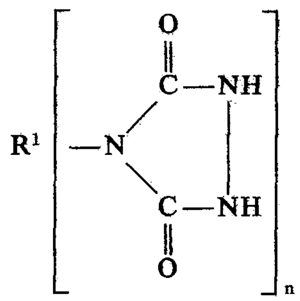

(V)

wherein

$R^1$ and n have the meaning indicated in formula I

is reacted with at least the equivalent amount of epihalohydrin or $\beta$-methylene epihalohydrin, optionally in the presence of a catalyst, to give the corresponding poly-(halohydrins) of the 1,2,4-triazolidine-3,5-dione of the formula indicated above and the resulting poly-halohydrins are then converted into 1,2,4-glycidyl-triazolidine-3,5-diones of the formula I with a hydrogen-halide-liberating agent.

6. Process for the preparation of glycidyl-1,2,4-triazolidine-3,5-diones according to Claim 5, characterised in that the epihalohydrin used is epichlorohydrin.

7. Process according to Claim 5, characterised in that 1.2 to 20 mol of epichlorhydrin are used per NH group of the 1,2,4-triazolidine-3,5-diones of the formula V.

**Revendications**

1. Glycidyl-1,2,4-triazolidine-3,5-diones de formule générale I

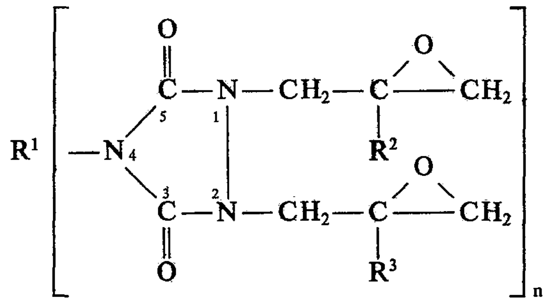

(I)

dans laquelle

$R^1$ est un reste aliphatique en $C_1$ à $C_{30}$ linéaire ou ramifié monovalent à pentavalent, un reste cycloaliphatique en $C_5$ à $C_{21}$ monovalent à pentavalent, un reste aliphato-aromatique en $C_7$ à $C_{17}$ monovalent à pentavalent ou un reste aromatique en $C_6$ à $C_{21}$ monovalent à pentavalent les restes mentionnés ci-dessus peuvent être substitués par au moins un groupe alkoxycarbonyle à groupe alkoxy en $C_1$ à $C_4$, CN, $NO_2$, un groupe alkylmercapto à groupe alkyle en $C_1$ à $C_4$, un groupe dialkylamino ayant 1 à 6 atomes de carbone dans chaque groupe alkyle, un halogène et, dans le cas des restes aromatiques, par au moins un groupe alkyle en $C_1$ à $C_4$ en plus des substituants mentionnés ci-dessus, en outre les restes aliphatiques mentionnés ci-dessus peuvent être interrompus par un ou plusieurs atomes d'oxygène ou atomes d'azote tertiaires, les restes cycloaliphatiques polycycliques, les restes aliphatoaromatiques polycycliques et les restes aromatiques polycycliques mentionnés ci-dessus peuvent être interrompus par au moins un atome d'oxygène ou un atome d'azote tertiaire ou par au moins un groupe alkylène ayant 1 à 4 atomes de carbone ou par au moins un groupe sulfonyle

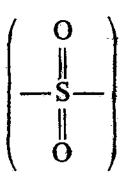

$R^2$ et $R^3$ sont égaux ou différents et représentent un atome d'hydrogène ou un groupe méthyle et
n est un nombre de 1 à 5.

2. Glycidyl-1,2,4-triazolidine-3,5-diones suivant la revendication 1, caractérisées en ce que $R^2$ et $R^3$ représentent l'hydrogène et $R^1$ n'est pas substitué.

3. Glycidyl-1,2,4-triazolidine-3,5-diones suivant la revendication 1, caractérisées en ce que $R^2$ et $R^3$ représentent l'hydrogène et $R^1$ est l'un des restes

a) $C_nH_{2n+1}$— n = 1 à 18

b) —$(CH_2)_m$— m = 2 à 12

c) 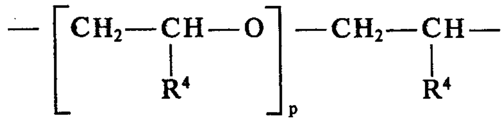 $R^4$ = H, $CH_3$; p = 1–9

d) 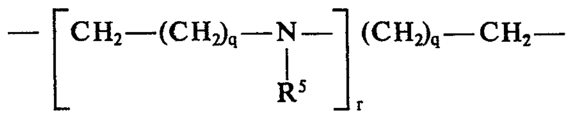 $R^5$ = $C_1-C_4$-Alkyle; q = 1–2; r = 1–4

e) 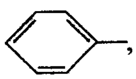, 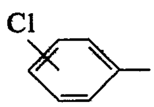

f) 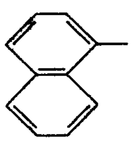

g) 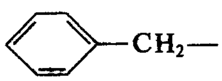

h) 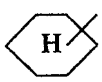

i) 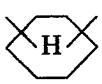

j) 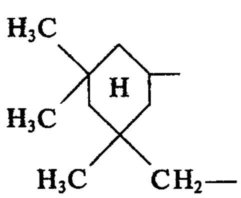

k) 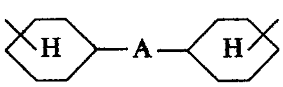 A = alkylène en $C_1-C_4$, O, $-N(CH_3)-$;

l) 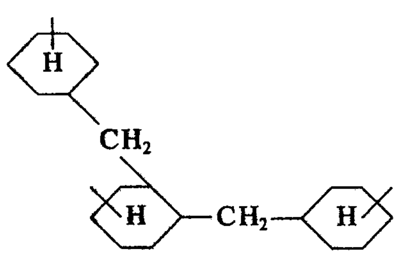

m) 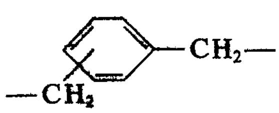

n) 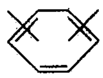

o) 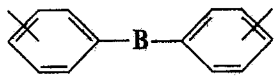

B = Alkylène en $C_1-C_4$, O, $-N(CH_3)-$;

p) 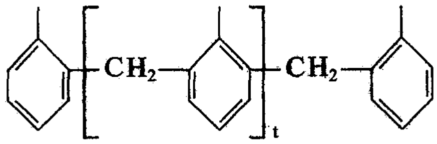

t = 1 à 3

q) $C_4H_9-O-CH_2-CH_2-CH_2-$

r) $-(CH_2)_3-O-(CH_2)_{\overline{2\ bis\ 4}}O-(CH_2)_3-$

s) 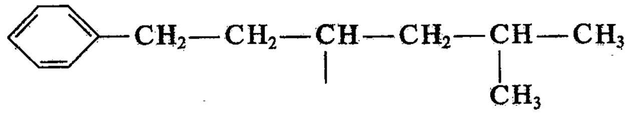

4. Glycidyl-1,2,4-triazolidine-3,5-diones suivant la revendication 1, caractérisées en ce que l'indice d'époxyde a une valeur de 0,2 à 0,91.

5. Procédé de production de 1,2,4-glycidyltriazolidine-3,5-diones suivant la revendication 1, caractérisé en ce qu'on fait réagir une 1,2,4-triazolidine-3,5-dione de formule V

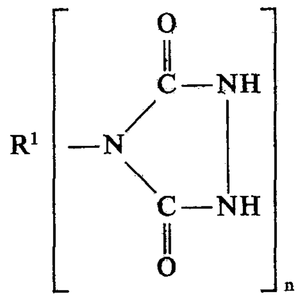

(V)

dans laquelle

$R^1$ et n ont la définition indiquée dans la formule I,

avec au moins la quantité équivalente d'épihalogénhydrine ou de $\beta$-méthylépihalogénhydrine, éventuellement en présence d'un catalyseur, pour former les poly(halogénhydrines) correspondantes de la 1,2,4-triazolidine-3,5-dione de formule indiquée ci-dessus que l'on transforme ensuite avec un agent libérant un halogénure d'hydrogène en 1,2,4-glycidyltriazolidine-3,5-diones de formule I.

6. Procédé de production de glycidyl-1,2,4-triazolidine-3,5-diones suivant la revendication 5, caractérisé en ce qu'on utilise l'épichlorhydrine comme épihalogénhydrine.

7. Procédé suivant la revendication 5, caractérisé en ce qu'on utilise, par groupe NH des 1,2,4-triazolidine-3,5-diones de formule V, 1,2 à 20 moles d'épichlorhydrine.